# EUROPEAN PATENT APPLICATION

(11) **EP 3 158 974 A1**
(43) Date of publication of application: **26.04.2017**
(21) Application number: 15810141.0
(22) Date of filing: 27.05.2015
(51) Int. Cl.: A61F 2/16

(54) **INTRAOCULAR LENS**

(30) Priority: 20.06.2014 JP 2014127311
(71) Applicant: Chukyo Medical Co., Inc., Aichi 456-0032 (JP)
(72) Inventor: ICHIKAWA, Kazuo, Nagoya-shi Aichi 465-0012 (JP); YOSHIDA, Norihiko, Nagoya-shi Aichi 468-0069 (JP)
(74) Representative: TBK
(86) International application number: PCT/JP2015/065170
(87) International publication number: WO 2015/194335

(57) **Abstract**

An intraocular lens (1) that comprises a lens (2) and a pair of loop-shaped haptics (3) formed in both sides of the lens, each of said haptics (3) being provided with a hooking member (30) at the top end thereof. In the case of, for example, implanting the intraocular lens (1) in an eye in such a manner that the top end of the haptic (3) is inserted into the scleral tunnel, the hooking member (30) hooks to somewhere within the scleral tunnel. Thus, the haptic (3) is strongly restrained in the scleral tunnel, which ensures the fixation of the intraocular lens. Namely, provided is an intraocular lens that is designed so that the top end of a supporting part of the intraocular lens can be kept outside or inside of the sclera without stitching.

## Description

### TECHNICAL FIELD

The present invention relates to an intraocular lens.

### BACKGROUND ART

In the related art, as a treatment for a cataract of an eye, a surgical operation is widely performed in which a clouded crystal lens of a patient is extracted and an intraocular lens (IOL) is inserted into an eye. For example, Patent Document 1 below proposes an intraocular lens which is inserted into an eye and is sewn to a ciliary body after a crystal lens is extracted during a treatment of a cataract.

In the technique disclosed in Patent Document 1, the intraocular lens is sewn to the ciliary body (an intraocular lens sewing method). On the contrary, a method of fixing the intraocular lens into the sclera without sewing (an intraocular lens inside sclera fixing method) is also proposed. In this technique, for example, when a portion near a front end of a haptic portion (a support portion) of the intraocular lens is inserted into a tunnel-shaped hole portion formed in the sclera in the circumferential direction, the insertion portion of the haptic portion is tightened by the tunnel so that the front end just stays inside the tunnel. Accordingly, the intraocular lens is supported without sewing.

According to the intraocular lens inside sclera fixing method, there is no need to learn an intraocular lens sewing technique necessary for the intraocular lens sewing method. Further, when this technique is used, the intraocular lens can be disposed at a center position of the eye with high accuracy and thus the lens fixing stability (particularly, the stability of the lens in the axial direction) is improved. Accordingly, there is an effect that the lens is not easily inclined.

Further, Patent Document 2 below proposes an intraocular lens in which a portion near a front end of a haptic portion is folded. If the folded portion of the haptic portion is inserted into the sclera when the intraocular lens having such a shape is fixed into the sclera without sewing, the haptic portion is more strongly held inside the sclera by the elastic restoration force of the folded portion and thus the stability of the intraocular lens is improved. In the same cited document, an embodiment is shown in which the folded portion protrudes toward the outside of the sclera. In this embodiment, when a force of drawing the haptic portion into the eye is exerted, the folded portion is hooked to the outer face of the sclera. Thus, the intraocular lens is stably held without sewing.

### CITATION LIST

### PATENT DOCUMENTS

Patent Document 1: JP 2792588 B2
Patent Document 2: JP 5398092 B1

### SUMMARY OF THE INVENTION

### PROBLEM TO BE SOLVED BY THE INVENTION

For example, when the front end of the haptic portion protrudes toward the outside of the sclera, there is a possibility that the haptic portion may be hooked to the outer face of the sclera even in a shape other than the folded portion. Further, even when the front end of the haptic portion is held inside the sclera, there is a possibility that the haptic portion can be effectively held even in a shape other than the folded portion. Further, even when the front end of the haptic portion is held by the outside or the inside of the sclera, there is a possibility that the haptic portion may be more effectively held even in a new embodiment with the folded portion. With such a development, a technique of supporting the intraocular lens into the eye can be further improved.

Further, this problem is not limited to the intraocular lens (IOL) which is implanted into the eye after the crystal lens is removed due to the cataract or the like, and also occurs in an intraocular contact lens (ICL) which is implanted into the eye in order to correct eyesight without removing the crystal lens. That is, there is a demand to improve the support performance inside the eye as described above even in the intraocular contact lens. Furthermore, the intraocular lens to be described later includes the intraocular contact lens.

Here, the invention is made in view of the above-described circumstances and an object of the invention is to provide an intraocular lens capable of causing a portion near a front end of a support portion thereof to be held outside or inside a sclera without sewing.

### MEANS FOR SOLVING PROBLEM

In order to solve the above-described problems, according to the invention, there is provided an intraocular lens including: a lens portion which has a lens function and is disposed in a posterior chamber in relation to an iris inside an eye; and a support portion which extends from a side limbus of the lens portion to have an outward arc shape in a radial direction of a visual axis and of which a front end is inserted toward a sclera so that the lens portion is fixed to the posterior chamber in relation to the iris inside the eye, wherein a portion inserted toward the sclera in the support portion includes a hook portion protruding from a side face of the support portion to be hooked to a part of the sclera. Accordingly, since the support portion is provided with the hook portion, the hook portion is hooked to a part of the sclera and thus the intraocular lens is stably supported inside the eye.

Further, the hook portion may have a protrusion shape protruding to be hooked to a part of the sclera when a force of drawing the support portion toward a root of the support portion is exerted. Accordingly, since the hook portion having the protrusion shape protruding toward a part of the sclera to be hooked thereto is effectively hooked to the sclera, the intraocular lens is stably supported inside the eye.

Further, the support portion may be inserted from a ciliary sulcus toward the sclera. Accordingly, the intraocular lens can be stably supported while the support portion is inserted from the ciliary sulcus to support the intraocular lens.

Further, the support portion may be inserted from a ciliary flat portion toward the sclera. Accordingly, the intraocular lens can be stably supported while the support portion is inserted from the ciliary flat portion to support the intraocular lens.

### BRIEF DESCRIPTION OF DRAWINGS

Fig. 1 is a diagram illustrating an embodiment of an intraocular lens of the invention;
Fig. 2 is an enlarged view illustrating a first example of a hook portion;
Fig. 3 is a cross-sectional view illustrating the first example of the hook portion;
Fig. 4 is a cross-sectional view illustrating a second example of the hook portion;
Fig. 5 is a cross-sectional view illustrating a third example of the hook portion;
Fig. 6 is a perspective view illustrating a fourth example of the hook portion;
Fig. 7 is a perspective view illustrating a fifth example of the hook portion;
Fig. 8 is a cross-sectional view illustrating the fifth example of the hook portion;
Fig. 9 is a diagram illustrating a sixth example of the hook portion (an example of a removable hook portion);
Fig. 10 is a diagram illustrating a first example of a state where the intraocular lens has been implanted into an eye;
Fig. 11 is a diagram illustrating a first example of a state where the intraocular lens is being implanted into an eye;
Fig. 12 is a diagram illustrating a second example of a state where the intraocular lens is being implanted into an eye;
Fig. 13 is a diagram illustrating a second example of a state where the intraocular lens has been implanted into an eye;
Fig. 14 is a diagram illustrating an embodiment including a folded portion of the intraocular lens of the invention;
Fig. 15 is an enlarged view illustrating first examples of a hook portion and a folded portion;
Fig. 16 is a diagram illustrating a third example of a state where the intraocular lens is being implanted into an eye;
Fig. 17 is a diagram illustrating second examples of the hook portion and the folded portion;
Fig. 18 is a diagram illustrating third examples of the hook portion and the folded portion (an example of a removable hook portion);
Fig. 19 is a diagram illustrating fourth examples of the hook portion and the folded portion;
Fig. 20 is a diagram illustrating fifth examples of the hook portion and the folded portion;
Fig. 21 is a diagram illustrating a third example of a state where the intraocular lens has been implanted into an eye;
Fig. 22 is a diagram illustrating sixth examples of the hook portion and the folded portion;
Fig. 23 is a diagram illustrating seventh examples of the hook portion and the folded portion;
Fig. 24 is a diagram illustrating eighth examples of the hook portion and the folded portion;
Fig. 25 is a diagram illustrating ninth examples of the hook portion and the folded portion;
Fig. 26 is a diagram illustrating tenth examples of the hook portion and the folded portion;
Fig. 27 is a diagram illustrating eleventh examples of the hook portion and the folded portion;
Fig. 28 is a diagram illustrating a seventh example of the hook portion;
Fig. 29 is a diagram illustrating an eighth example of the hook portion;
Fig. 30 is a diagram illustrating a fourth example of a state where the intraocular lens has been implanted into an eye; and
Fig. 31 is a diagram illustrating a fifth example of a state where the intraocular lens has been implanted into an eye.

### MODE(S) FOR CARRYING OUT THE INVENTION

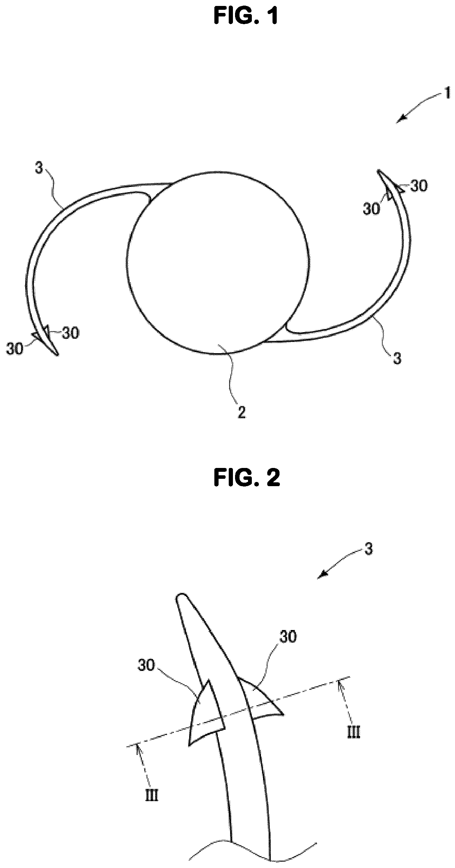

Hereinafter, embodiments of the invention will be described with reference to the drawings. First, Figs. 1 to 3 are diagrams illustrating an embodiment of an intraocular lens 1 of the invention. Fig. 1 is a front view illustrating the entire intraocular lens 1 (a description for a direction such as a front face or a lateral face indicates a direction (a lateral face, a front face, or the like) in a face (or an eye) of a patient having an intraocular lens fixed into an eye).

The intraocular lens 1 mainly includes a lens 2 and a haptic portion 3. For example, after a crystal lens clouded by a cataract is removed (entirely or partially) from an eye of a patient, the lens 2 is disposed in a posterior chamber of an eye (or an eye posterior chamber, that is, an area behind an iris) and conducts a lens function of the removed crystal lens.

The haptic portion 3 is a portion which supports the lens 2 to the posterior chamber. In Fig. 1 and the like, the haptic portion 3 is provided at two positions of the side limbus of the lens 2 to extend in a pair of loop shapes (or arc shapes) and to have a line symmetrical shape with respect to a visual axis (the center axis of the lens 2). In the example of Fig. 1, the haptic portion 3 is formed in a curved shape which extends outward in the radial direction with respect to the visual axis and also extends in the circumferential direction as it goes toward a front end side.

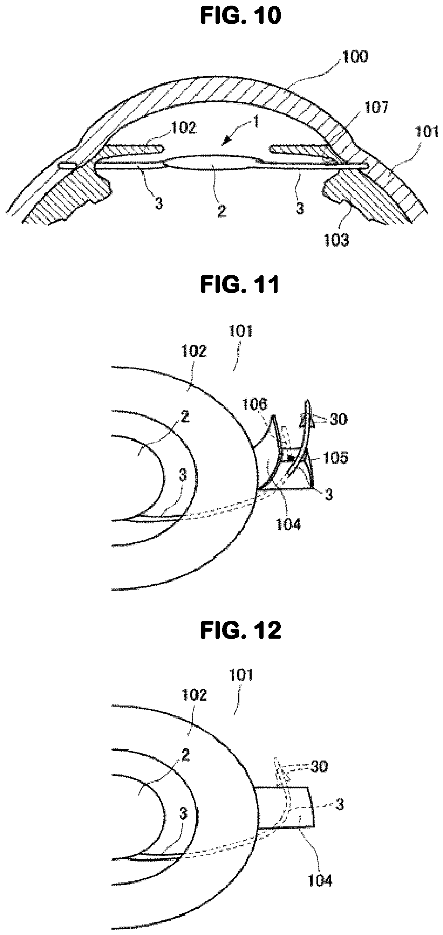

As illustrated in Fig. 10 and the like to be described later, a shape is desirable in which the haptic portion 3 enters, for example, the substantially same plane as the lens 2 when the haptic portion 3 is viewed from the lateral side. Alternatively, the haptic portion 3 may be formed to extend toward a front side within the eye and to obliquely extend toward the posterior chamber from the side limbus of the lens 2 when the haptic portion is viewed from the lateral side.

The intraocular lens 1 of the invention may be formed of the same material (for example, PMMA or the like) as the intraocular lens of the related art. Accordingly, the intraocular lens 1 has flexibility (elasticity and softness). For example, the intraocular lens can be rounded in a bar shape and can be inserted into the eye by an injector or the like. The haptic portion 3 may be formed of, for example, a resin material to be integrally molded with the lens 2. Alternatively, the haptic portion 3 may be formed separately from the lens 2 and may be bonded (adhered) thereto.

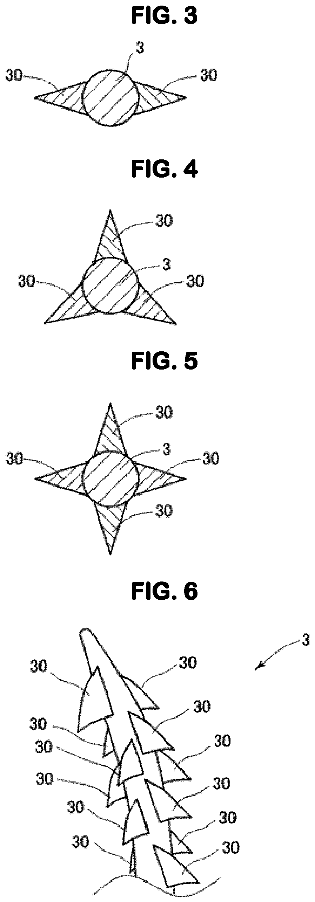

As a main part of the invention, the intraocular lens 1 includes a hook portion 30 which is provided in the vicinity of the front end of the haptic portion 3. As will be described later, the hook portion 30 is a portion which hooks the intraocular lens 1 to a part of a sclera in order to guide the intraocular lens into the eye. In the examples of Figs. 1 to 3, two hook portions 30 are provided in the vicinity of the front end of the haptic portion 3 to protrude in a protrusion shape from a side face thereof. In the example of Fig. 3, two hook portions 30 are arranged equally in the circumferential direction (at symmetrical positions with respect to the axis line of the haptic portion 3).

In the examples of Figs. 2 and 3, the hook portion 30 has a substantially triangular shape in both lateral and cross-sectional views. That is, one hook portion 30 is a three-dimensional object having four triangular faces. In this example, the hook portion 30 may be integrally formed with, for example, the haptic portion 3 so as not to be separable from the haptic portion 3. The position of the hook portion 30 (a position of the haptic portion 3 in the axial direction or the circumferential direction) may be disposed at an appropriate position to be hooked to the sclera to be described later.

The arrangement of the hook portions 30 illustrated in Fig. 3 is merely an example, but may be formed as in, for example, Figs. 4 and 5. In the example of Fig. 4, three hook portions 30 are equally arranged in the circumferential direction. Further, in the example of Fig. 5, four hook portions 30 are equally arranged in the circumferential direction. Furthermore, the hook portions 30 may be arranged as illustrated in Fig. 6. In the example of Fig. 6, a plurality of (plural) hook portions 30 are arranged to be distributed to at various positions in the axial direction and the circumferential direction.

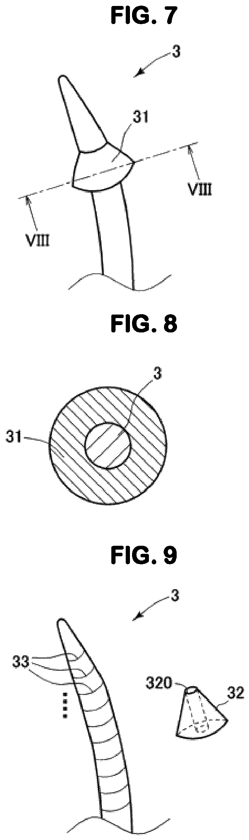

Furthermore, the hook portion 30 may be formed in a shape illustrated in Figs. 7 and 8. In this example, a tapered (conical) hook portion 31 is formed around the haptic portion 3 to be tapered toward the front end. The invention is not limited to the above-described example. For example, the hook portion of the invention may protrude from the side face of the haptic portion 3 in any shape. However, at that time, it is desirable that the hook portion protrude in an appropriate direction to be hooked to the sclera as described later.

In the above-described example, the protrusion shapes of the hook portions 30 and 31 are formed so that a protrusion is formed obliquely in the opposite direction from the front end of the haptic portion 3 (as a different expression, the protrusion directions of the hook portions 30 and 31 are inclined toward the root of the haptic portion 3 in relation to a direction perpendicular to the side face of the haptic portion 3). Each of the protrusion shapes of the hook portions 30 and 31 may be formed in a sharp shape forming at an acute angle toward the root of the haptic portion 3.

In the above-described example, the hook portions 30 and 31 may be integrally formed with the haptic portion 3, but the invention is not limited to the embodiment. For example, the hook portion 30 may be formed to be attachable to and detachable from the haptic portion 3. The example is illustrated in Fig. 9.

In the example of Fig. 9, a conical hook portion 32 without a front end is formed separately from the haptic portion 3 and is attached to the haptic portion 3. The hook portion 32 is formed so that a hole portion 320 penetrates the inside in the axial direction and the haptic portion 3 is inserted thereinto. The operation may be performed, for example, before an operator performs an intraocular lens implantation operation.

In order to appropriately perform an operation of integrating the hook portion 32 with an appropriate position of the haptic portion 3, a plurality of scale marks 33 are shown in the haptic portion 3 of Fig. 9. By using the scale mark 33 as a reference, the hook portion 32 can be disposed at an appropriate position of the haptic portion 3. As for the integration of the hook portion 32 and the haptic portion 3, for example, the diameter of the hole portion 320 is set to be substantially equal to the diameter of the haptic portion 3 and the hook portion 32 is simply fitted (press-inserted) thereinto for the purpose of the sufficiently reliable integration.

Fig. 10 illustrates an example in which the intraocular lens 1 is implanted (attached) into the eye. Then, an example of a surgical operation is illustrated in Figs. 11 and 12. With reference to these drawings, an example of the implantation procedure will be described.

The intraocular lens 1 of the invention is fixed into the sclera of the posterior chamber (the eye posterior chamber) of the eye (without sewing) after the crystal lens clouded by the cataract is entirely or partially extracted. As illustrated in Fig. 10, a pair of the haptic portions 3 formed at the left and right sides of the lens 2 while the lens 2 is disposed at a position behind the iris 102 are buried inside a sclera 101. Fig. 10 illustrates a case where the crystal lens is entirely extracted.

The implantation procedure of the intraocular lens of the invention is similar to, for example, the document (Journal of Cataract and Refractive Surgery, vol. 34, pp.1433-1438 (2008)) of Amar Agarwal and an example thereof is described as below.

After a conjunctivitis is cut out, as illustrated in Fig. 11, a sclera half layer valve 104 which is a valve having a substantially half thickness of the sclera is formed at a position adjacent to a limbus of a cornea 100 of the sclera 101. A position where the sclera half layer valve 104 is formed is set to two symmetrical positions with respect to the visual axis at a position adjacent to the limbus of the surface of the sclera. In Fig. 11, one of two sclera half layer valves 104 formed in this way is illustrated.

Then, the sclera is cut out toward, for example, the center of the eye ball (or toward the ciliary sulcus or the ciliary flat portion) from one position of the lower layer of the sclera half layer valve 104 of the sclera 101. Further, a tunnel 106 is formed inside the sclera in a direction substantially parallel to the limbus, that is, the circumferential direction of the visual axis. That is, the tunnel is formed at a position 105 as an inlet to have, for example, a substantially half depth of the sclera thickness from the surface of the sclera at the perforated side wall of the sclera half layer valve 104. The tunnel 106 may be formed as an outlet to penetrate the surface of the sclera while having an appropriate length (for example, 2 mm to 3 mm) inside the sclera.

Then, the intraocular lens 1 is inserted from, for example, a notch formed in the cornea 100 into the eye by a predetermined injector or the like while the intraocular lens is rounded in a bar shape. For this insertion, one haptic portion 3 is located at the leading side and the other haptic portion 3 is located near the posterior chamber. Even when the leading haptic portion 3 and the lens 2 are inserted into the eye, the haptic portion 3 near the posterior chamber is partially exposed to the outside from the notch of the cornea 100.

As illustrated in Fig. 11, the front end of the leading haptic portion 3 inserted into the eye is drawn out from the posterior chamber through the sclera notch by, for example, an implement such as a 25-g forceps so that the front end thereof is exposed to the outside of the eye from the lower portion of the sclera half layer valve 104 while the sclera half layer valve 104 is opened. At that time, a portion provided with the hook portion 30 is included in the exposed haptic portion 3.

Then, the haptic portion 3 which is exposed to the outside of the eye is inserted into the tunnel 106 formed inside the sclera. By the above-described procedure, for example, an implement such as a 25-g forceps may be used as described above. The above-described processes are also performed on one haptic portion 3 located at one symmetrical position with respect to the visual axis. Accordingly, the haptic portion is received into the tunnel 106 inside the sclera along with the hook portion 30 while the front ends of two haptic portions 3 are symmetrical to each other with respect to the visual axis.

When the above-described procedure ends, two sclera half layer valves 104 are closed. At this time, for example, fiblin glue is applied to (the rear face of) the sclera half layer valve 104 or the opposite face thereof and the sclera half layer valve 104 is adhered to the sclera 101. The above-described method is an example of a main procedure of the method of fixing the intraocular lens into the sclera without sewing. When the tunnel 106 inside the sclera tightens the haptic portion 3 by the above-described procedure, the haptic portion 3 is restricted and held inside the tunnel 106 in the sclera and further the hook portion 30 is hooked to the inner wall of the tunnel 106 in the sclera, whereby the intraocular lens 1 is fixed into the eye without sewing.

Alternatively, the intraocular lens 1 of the invention may be implanted into the eye by the technique disclosed in the document (Journal of Cataract and Refractive Surgery, vol. 33, pp. 1851-1854 (2007)) of Gabor B. Scharioth.

In this technique, the sclera half layer valve 104 is not formed and the sclera is cut out from the surface of the sclera 101 toward the posterior chamber. Then, the tunnel 106 inside the sclera (the hole portion) is formed in a direction substantially parallel to the limbus, that is, the circumferential direction of the visual axis to have an appropriate length (for example, 2 to 3 mm) from a position located at the substantially half thickness of the sclera thickness in the sclera notch. Here, the tunnel penetrates the sclera surface as an outlet.

Then, as described above, the haptic portion 3 is inserted into the tunnel 106 inside the sclera along with the hook portion 30. In this case, the sclera notch may be sewn if necessary after the front end is inserted into the tunnel 106 inside the sclera. Furthermore, a method of implanting the intraocular lens 1 of the invention into the eye is not limited to the methods of two documents described above, and all methods of fixing the intraocular lens into the sclera can be used.

If the intraocular lens 1 is implanted into the eye as described above, the hook portion 30 is strongly hooked to the sclera 101 when a force of drawing the haptic portion 3 toward the root is exerted and thus the haptic portion strongly resists the drawing force. At that time, it is effective when the protrusion direction of the hook portion 30 is set to a direction toward the root of the haptic portion 3 as described above.

From the past, in the method of fixing the intraocular lens into the sclera without sewing, the sclera strongly tightens the haptic portion to effectively hold the intraocular lens. However, in the invention, since the effect of the hook portion 30 is additionally provided, the intraocular lens is held further stably and hence the separation or the positional deviation (the rotation or the like) of the intraocular lens is effectively suppressed. Further, when the haptic portion 3 is inserted into the sclera 101 in the protrusion direction of the hook portion 30, the hook portion 30 can be smoothly inserted into the sclera while not being hooked thereto. That is, the protrusion direction of the hook portion 30 is a direction in which the hook portion 30 is hooked into the sclera when a force of drawing the haptic portion 3 toward the root is exhibited and the hook portion 30 is not hooked into the sclera when a force of inserting the haptic portion 3 into the sclera is exhibited.

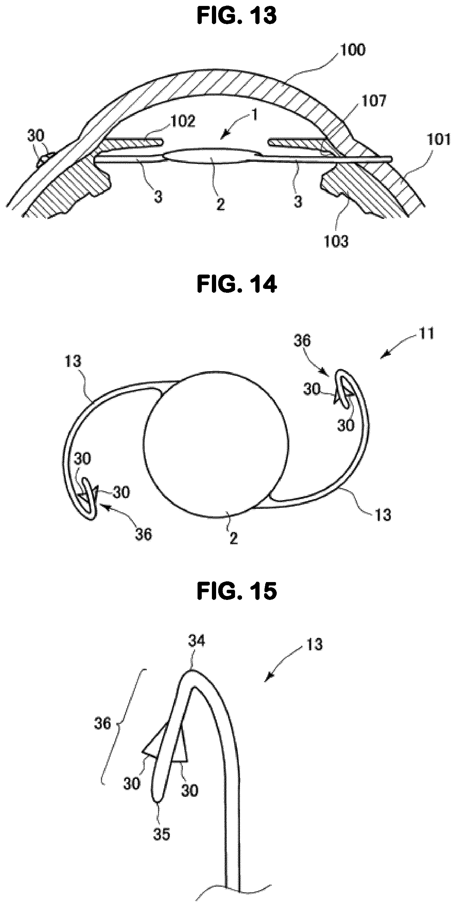

In the description above, a case has been described in which the intraocular lens of the invention is fixed into the sclera 101. However, the intraocular lens of the invention is not limited to a case where a portion to be hooked by the hook portion 30 is the inside of the sclera 101, and the hook portion may be hooked to the outer face of the sclera 101. The example is illustrated in Fig. 13.

In this example, the front end of the haptic portion 3 is drawn to the outside of the sclera 101 while the procedure of forming the sclera half layer valve 104 or the tunnel 106 inside the sclera is omitted from the above-described implantation procedure. At that time, a portion provided with the hook portion 30 is also drawn to be exposed to the outside of the sclera 101. Accordingly, the vicinity of the front end of the loop-shaped haptic portion 3 is located so as to smoothly follow the outer face of the sclera 101. Accordingly, the hook portion 30 is hooked to the outer face of the sclera 101.

Thus, for example, even when a force of drawing the haptic portion 3 toward the root is exhibited in this state, the hook portion 30 is hooked to the outer face of the sclera 101. Accordingly, the haptic portion 3 of the intraocular lens 1 can be further stably fixed into the eye.

In the intraocular lens of the invention, the folded portion may be provided near the front end of the haptic portion 3 as in Patent Document 2 in addition to the hook portion 30. An example of the embodiment will be described below with reference to Fig. 14 and the like.

An intraocular lens 11 illustrated in Figs. 14 and 15 includes a folded portion 36 which is folded toward the front end of the haptic portion 13 to extend in the opposite direction. The folded shape of the folded portion 36 may be formed to have a corner portion or may be folded in a curve shape without a corner portion.

As described above, the haptic portion 13 has elasticity. For example, in a natural state (that is, a state where no external force is exhibited), the folded portion 36 may be separated from the other portions as illustrated in Fig. 14 and the like. In this case, when the folded portion 36 is folded (that is, the folded portion 36 is formed to overlap the other haptic portion 13 in a linear shape), an elastic restoration force is exerted so that the folded portion 36 is separated from the other portions as illustrated in Fig. 14. Alternatively, a shape illustrated in Fig. 14 may be maintained so that the folded portion of the front end of the haptic portion 13 does not have enough elasticity.

As illustrated in Fig. 15, the folded portion 36 is folded at a bent portion 34 and a front end 35 of the haptic portion 13 is directed in a direction opposite to the extension direction of the haptic portion 3. Then, the hook portion 30 is disposed at the intermediate portion of the folded portion 36. The hook portion 30 may have the above-described shape.

When the haptic portion is inserted into the tunnel 106 inside the sclera while the folding angle of the bent portion 34 is not opened too much, an angle may be set such that the folded portion 36 and the other haptic portion 13 are inserted into the tunnel 106 inside the sclera while the bent portion 34 is located at the leading position.

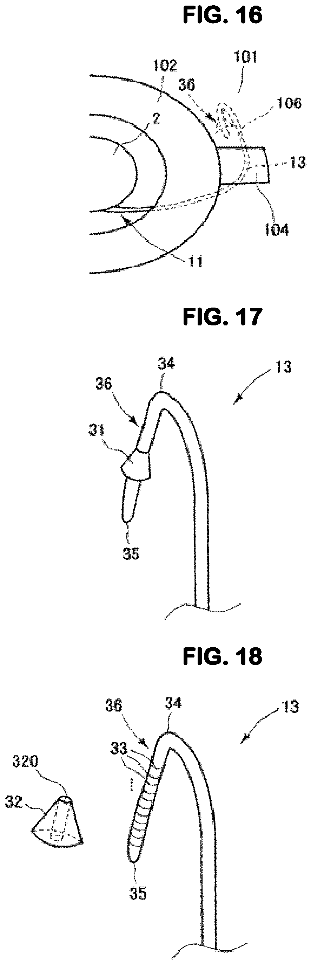

An example in which the intraocular lens 11 is implanted into the eye is illustrated in Fig. 16. This example shows an embodiment in which the haptic portion 13 is inserted into the tunnel 106 inside the sclera. The intraocular lens 11 may be implanted similarly to the implantation of the intraocular lens 1, but in the embodiment in which the haptic portion 13 is inserted into the tunnel 106 inside the sclera, the haptic portion is inserted while the bent portion 34 is located at the leading position.

In order to enable the insertion, the folding angle of the bent portion 34 may be set to an angle in which the bent portion is inserted into the tunnel 106 inside the sclera along with a part of the haptic portion 13 other than the folded portion. Then, the length of the folded portion may be set to a length in which the entire folded portion is received into the tunnel-shaped hole portion formed inside the sclera along with a part of the haptic portion 13 other than the folded portion.

The folded portion 36 is inserted until the front end 35 is received into the tunnel 106 inside the sclera, that is, the entire folded portion 36 is received into the tunnel 106 inside the sclera. Accordingly, the bent portion 34 and the front end 35 of the haptic portion are also received into the tunnel 106 inside the sclera.

In the embodiment, the folded portion 36 and the hook portion 30 have the following effects. If the bent portion 34 has enough elasticity, the folded portion 36 may be disposed to overlap the other portion of the haptic portion 3 in a linear shape when the folded portion 36 is inserted into the tunnel 106 inside the sclera according to the above-described procedure. When the folded portion 36 is received into the tunnel 106 inside the sclera, the folded portion is opened again by the elastic restoration force thereof and is pressed against the inner wall of the tunnel 106 inside the sclera.

Accordingly, the inner wall of the tunnel 106 inside the sclera is stretched, but the inner wall is stretched to a certain degree by the strength of the sclera so that the front end including the folded portion 36 is pushed back from the outward. Accordingly, a balance between the pushing force and the pushing-back force is obtained. Due to the tension state, the front end including the folded portion 36 hardly moves inside the tunnel 106 inside the sclera.

When a force is exerted so that the haptic portion 3 is separated from the inlet 105 of the tunnel 106 inside the sclera, the front end 35 is hooked to a certain portion of the inner wall of the tunnel 106 inside the sclera. Further, the hook portion 30 is also hooked to a certain portion of the tunnel inside the sclera. With such a complex effect, the haptic portion 3 is strongly restricted in the tunnel 106 inside the sclera so as not to be separated therefrom. By the above-described effect, the intraocular lens 11 is reliably fixed into the eye.

Additionally, the bent portion 34 may not have elasticity in which the folded portion 36 is folded. In that case, the folded portion 36 is inserted into the tunnel 106 inside the sclera so that the tunnel 106 inside the sclera is enlarged in such a shape while not being folded.

Even in this case, when a force is exerted in which the folded portion 36 is pressed against the inner wall of the tunnel 106 inside the sclera so that the folded portion 36 is drawn out from the inlet 105, the front end of the folded portion 36 is hooked to a certain portion of the inner wall of the tunnel 106 inside the sclera and the hook portion 30 is also hooked to a certain portion of the inner wall of the tunnel 106 inside the sclera. Thus, since the movement of the haptic portion 13 in the tunnel 106 inside the sclera is strongly suppressed, the intraocular lens 11 is reliably fixed into the eye.

The hook portion 30 provided in the intraocular lens 11 may also have various shapes as illustrated in Figs. 3 to 6. That is, three or four hook portions 30 may be disposed (at equivalent positions) in the circumferential direction. Alternatively, the hook portions may be disposed to be distributed in the axial direction and the circumferential direction. Further, the hook portion 30 may have a shape illustrated in Figs. 7 and 8. That is, as illustrated in Fig. 17, the tapered (conical) hook portion 31 which is tapered toward the front end of the haptic portion 3 may be formed around the haptic portion. As described above, the hook portion of the intraocular lens 11 may protrude from the side face of the haptic portion 13 to have a certain shape. However, it is desirable that the hook portion protrude in an appropriate direction to be hooked to the sclera at that time.

Further, the hook portion 30 of the intraocular lens 11 may be attachable to and detachable from the haptic portion 13 as in Fig. 9. That is, as illustrated in Fig. 18, the conical hook portion 32 without the front end may be formed separately from the haptic portion 13 and may be attached to the folded portion of the haptic portion 13. The hook portion 30 is provided with the hole portion 320 which penetrates therein in the axial direction and the folded portion of the haptic portion 13 is inserted thereinto. The haptic portion 13 is provided with a plurality of scale marks 33 which are used as references so that the hook portion 30 is integrated with an appropriate position of the folded portion.

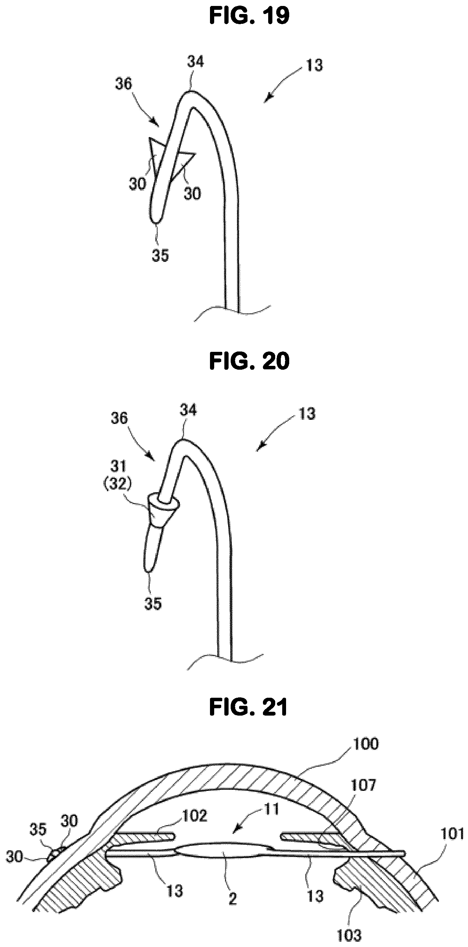

Next, the invention may have an appropriate configuration in which both the folded portion 36 and the hook portion 30 (31, 32) are provided and are hooked to the outer face of the sclera 101. The example is illustrated in Figs. 19 to 21. In this example, in the haptic portion 13 having a shape with the bent portion 34 as in Fig. 15 or 17, the hook portion 30 is disposed in the opposite direction of Fig. 15 or 17. That is, the hook portion 30 illustrated in Fig. 19 and the hook portion 31 (32) illustrated in Fig. 20 are formed so that a protrusion shape is obliquely directed upward in the drawing.

Fig. 21 illustrates a state where the intraocular lens 11 including the haptic portion 13 having such a shape is implanted into the eye so that the front end of the haptic portion 13 is inserted from the ciliary sulcus and is drawn to the outside of the sclera 101. In this example, the haptic portion 13 is drawn out until the bent portion 34 comes out of the sclera 101. For this configuration, the length of the haptic portion 13 or the position of the bent portion 34 is appropriately designed.

In the state of Fig. 21, the hook portion 30 is hooked to the outer face of the sclera 101. When a force of drawing the haptic portion 13 toward the root is exerted by the direction of the protrusion shape of the hook portion 30, the hook portion 30 is strongly hooked to the outer face of the sclera and resists the drawing force. Accordingly, the existence of the bent portion 34 also serves as a resistance for the drawing force. By such an effect, the separation or the positional deviation (the rotation or the like) of the intraocular lens 11 is effectively suppressed. Furthermore, the bending angle or the bending direction of the bent portion 34 may be set so that the side face of the folded portion 36 naturally contacts the outer face of the sclera 101 (or the side face is pressed against the outer face of the sclera 101) while the front end of the haptic portion 13 is drawn to the outside of the sclera 101.

Further, the embodiment of Fig. 21 may be modified to form the sclera half layer valve. That is, the sclera half layer valve is formed and the folded portion 36 of the haptic portion 13 is entirely drawn out while the sclera half layer valve is folded. Then, the sclera half layer valve is closed and sewn so that the entire drawn folded portion 36 is hidden. In this embodiment, since the haptic portion 13 is not visually recognized by the other persons after the implantation of the intraocular lens, the intraocular lens is very appropriate in appearance.

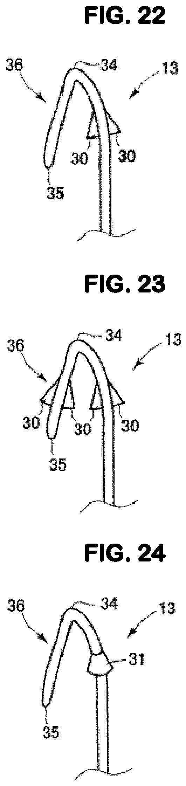

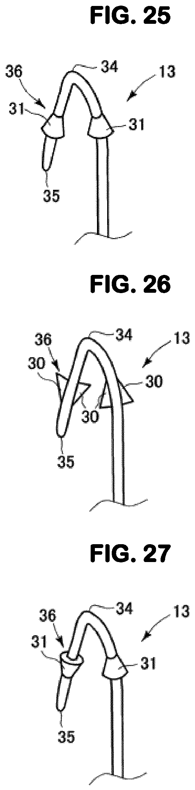

The embodiment in which the hook portion 30 (31, 32) is provided in the haptic portion 13 with the folded portion 36 is not limited to Figs. 15, 17, and the like. A different embodiment is illustrated in Fig. 22 and the like. Fig. 22 is an embodiment in which the hook portion 30 is provided near the root of the haptic portion 13 in relation to the bent portion 34. Fig. 23 illustrates an embodiment in which the hook portion 30 is provided near both the root and the front end of the haptic portion 13 in relation to the bent portion 34. Fig. 24 illustrates an embodiment in which the hook portion 31 is provided near the root of the haptic portion 13 in relation to the bent portion 34. Fig. 25 illustrates an embodiment in which the hook portion 31 is provided near both the root and the front end of the haptic portion 13 in relation to the bent portion 34.

Further, each of the hook portions 30 (31) of the embodiments illustrated in Figs. 22 to 25 is formed in a shape protruding obliquely downward. Each of the haptic portions 13 illustrated in Figs. 22 to 25 may be inserted into the sclera 101 while the bent portion 34 is located at the leading position as in Fig. 16. Accordingly, when a force which draws the haptic portion 13 toward the root is exerted as in Figs. 14 to 18, the hook portions 30 and 31 are hooked to the inside of the sclera and thus the intraocular lens is stably supported.

Next, Figs. 26 and 27 illustrate embodiments in which the protrusion direction of the hook portion 30 (31) near the front end 35 in relation to the bent portion 34 is changed to the oblique upward direction illustrated in Figs. 23 and 25. The haptic portions 13 illustrated in Figs. 26, 27, 22, and 24 may be disposed inside the eye so that the entire folded portion 36 is drawn to the outside of the sclera 101 similarly to Fig. 21. Alternatively, an embodiment may be employed in which the above-described sclera half layer valve is formed and the entire folded portion 36 is disposed therebelow.

Accordingly, when a force of drawing the haptic portion 13 toward the root is exerted, the bent portion 34 is hooked to the outer face of the sclera 101. Further, the hook portions 30 and 31 near the front end 35 in relation to the bent portion 34 are hooked to the outer face of the sclera and the hook portions 30 and 31 near the root in relation to the bent portion 34 are hooked to the inside of the sclera. By such an effect, the intraocular lens is stably supported inside the eye.

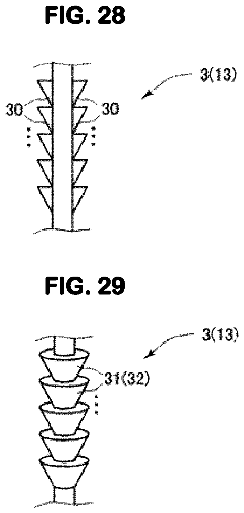

Additionally, all hook portions 30 may be disposed to be continuous in the axial direction of the haptic portion 3 (13) as illustrated in Fig. 28. Further, all hook portions 31 (32) may be also disposed to be continuous in the axial direction of the haptic portion 3 (13) as illustrated in Fig. 29. Of course, when a force of drawing the haptic portion 3 (13) toward the root is exerted in Figs. 28 and 29, the intraocular lens is implanted so that the hook portion 30 (31) is drawn upward in the drawing. By such an embodiment, the hooking effect at a plurality of positions can be reliably obtained.

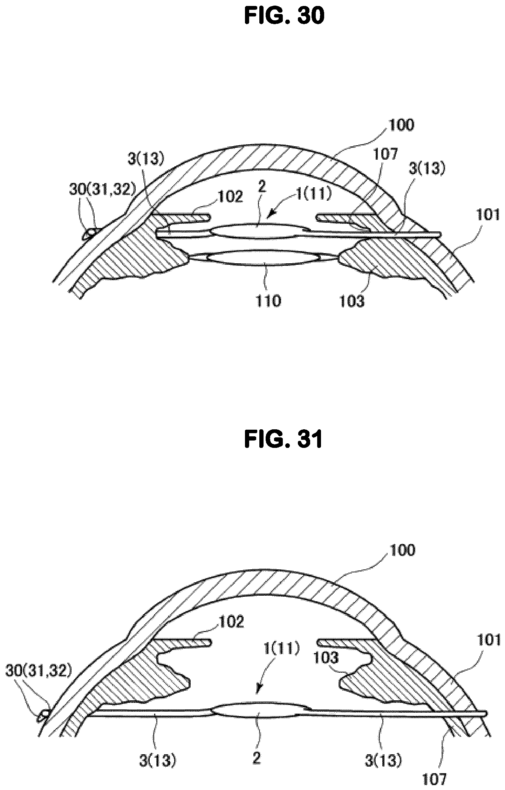

In the above-described embodiment, an example has been described in which the intraocular lens is implanted into the eye from which the crystal lens is removed, but the invention is not limited thereto. For example, an embodiment of the intraocular contact lens also exists. The example is illustrated in Fig. 30. In the embodiment illustrated in Fig. 30, a crystal lens 110 of the patient is not removed, and the intraocular lens 1 or 11 (the intraocular contact lens) is implanted into the front side (and the posterior chamber in relation to the iris 102). The intraocular lens 1 (11) of Fig. 30 may be any intraocular lens of the above-described example. The haptic portion 3 (13) of the intraocular contact lens 1 (11) is drawn to the outside of the sclera 101 from the ciliary sulcus as in the above-described example. Accordingly, since the position of the intraocular contact lens 1 (11) is stably fixed when the hook portion 30 (31, 32) is hooked to the outside of the sclera, the eyesight of the patient can be appropriately corrected. Furthermore, the embodiment of Fig. 30 may be modified so that the front end of the haptic portion 3 (13) is inserted into the tunnel inside the sclera as in Fig. 10.

In the above-described embodiment, the haptic portion 3 (13) is drawn from a ciliary sulcus 104 to the inside or the outside of the sclera 101, but the invention is not limited to the embodiment. A different embodiment is illustrated in Fig. 31. In this example, the haptic portion 3 (13) of the intraocular lens 1 (11) is drawn from the ciliary flat portion 107 to the outside of the sclera 101 after the crystal lens is removed. Accordingly, the hook portion 30 (31, 32) is hooked to the outside of the sclera 101 so that the haptic portion 3 (13) and the intraocular lens 1 (11) are stably positioned. In this way, the invention is suitable for the new implantation embodiment using the ciliary flat portion 107.

The above-described embodiment may be modified within the scope of claims. For example, in the above-described embodiment, two support portions are provided, but the invention is not limited thereto. For example, the number of the support portions may be arbitrarily set to three, four, five, six, or the like. Further, in the above-described example, the support portion is formed as a haptic shape (a loop shape), but may be formed in a shape other than the haptic shape. For example, the support portion may be formed in a leg shape extending from the limbus of the lens 2 outward in the radial direction of the visual axis or a shape of which the front end extends in the circumferential direction. Even when a plurality of hook portions are provided in the circumferential directions of the haptic portions 3 and 13, the number is not limited to two to four, but may be arbitrarily five, six, or the like.

Further, the folding direction of the folded portion 36 is set so that the folding portion is folded inward in the example of Fig. 1 (toward the center of the lens 2). However, the folding portion may be folded outward. Further, the folding portion may be folded in the other directions, that is, a direction intersecting the plane including the haptic portion 3 (or the lens 2).

### EXPLANATIONS OF LETTERS OR NUMERALS

- 1, 11: intraocular lens
- 2: lens (lens portion)
- 3, 13: haptic portion (support portion)
- 30, 31, 32: hook portion
- 34: bent portion
- 36: folded portion

## Claims

1. An intraocular lens comprising:
a lens portion which has a lens function and is disposed in a posterior chamber in relation to an iris inside an eye; and
a support portion which extends from a side limbus of the lens portion to have an outward arc shape in a radial direction of a visual axis and of which a front end is inserted toward a sclera so that the lens portion is fixed to the posterior chamber in relation to the iris inside the eye,
wherein a portion inserted toward the sclera in the support portion includes a hook portion protruding from a side face of the support portion to be hooked to a part of the sclera.

2. The intraocular lens according to claim 1,
wherein the hook portion has a protrusion shape protruding to be hooked to a part of the sclera when a force of drawing the support portion toward a root of the support portion is exerted.

3. The intraocular lens according to claim 1 or 2,
wherein the support portion is inserted from a ciliary sulcus toward the sclera.

4. The intraocular lens according to claim 1 or 2,
wherein the support portion is inserted from a ciliary flat portion toward the sclera.
